# EUROPEAN PATENT APPLICATION

(11) **EP 1 707 229 A1**
(43) Date of publication of application: **04.10.2006**
(21) Application number: 06006587.7
(22) Date of filing: 29.03.2006
(51) Int. Cl.: A61M 5/32, A61C 5/02, B21G 1/08

(54) **Liquid injection needle for jetting a liquid in a predetermined angle range and method of producing the same**

(30) Priority: 29.03.2005 JP 2005094503
(71) Applicant: Matsumoto Dental University, Nagano 399-0781 (JP); Mikuni Kogyo Co., Ltd., Okaya-shi Nagano 394-8520 (JP)
(72) Inventor: Kuroiwa, Akihiro c/o Matsumoto Dental University, Nagano 399-0781 (JP); Yamamoto, Akio c/o Matsumoto Dental University, Nagano 399-0781 (JP); Otogoto, Junichi c/o Matsumoto Dental University, Nagano 399-0781 (JP); Murakoso, Takaaki, Okaya-shi, Nagano 394-8520 (JP); Hasegawa, Hiroshi, Okaya-shi, Nagano 394-8520 (JP); Yamaguchi, Iwao, Okaya-shi, Nagano 394-8520 (JP)
(74) Representative: Vossius & Partner

(57) **Abstract**

A cannula (21) has a front part (21 c) in a direction of a center axis X with its forward end portion (23) closed. The front part has a plurality of nozzle orifices (24, 25, 26) each of which has an orifice axis Y intersecting the center axis X. Each of the nozzle orifices has a first diameter in a first direction parallel to the center axis X and a second diameter in a second direction perpendicular to the first direction. The first diameter ranges between 0.1 mm and 0.5 mm while the second diameter ranges between 0.1 mm and 0.3 mm so that a liquid in the cannula is jetted from the nozzle orifices in an angle range of 45 degrees towards the forward end portion with respect to the orifice axis.

## Description

This invention relates to a liquid injection needle element capable of jetting a liquid in a cannula or needle tube to the outside of the cannula and a method of producing the same.

In the dental practice or treatment, a tooth is often ground. After the tooth is ground, grinding chips left around the tooth sometimes enter a small gap at the bottom of the tooth or in a root canal of the tooth. Those grinding chips are removed by jetting a liquid (cleaning liquid) from a cannula.

In the dental treatment, the cannula is also used when a periodontal disease is treated. In this case, a forward end portion of the cannula is inserted into a periodontal pocket (a space between a tooth and a gingiva) which is an affected part of the periodontal disease and the liquid is jetted from the cannula.

Japanese Examined Patent Publication (JP-B) No. H06-73530 (Patent Document 1) (corresp. to US patent No. 4,993,941) discloses a dental irrigation needle including a needle hub engageable with a syringe and a cannula.

The cannula in the Patent Document 1 comprises a bendable metal and has a closed end. One or more nozzle orifices are formed on a lateral wall of the cannula at its front part so that a jet of liquid is delivered therethrough.

Japanese Unexamined Patent Application Publication (JP-A) No. 2003-62068 (Patent Document 2) discloses a liquid injection needle element including a cannula having a plurality of nozzle orifices formed at its front part so that a jet of liquid is delivered therethrough.

The cannula in the Patent Document 2 has flexibility and can be bent and shaped by an operator's hand into a widely or gently curved shape as a whole.

The cannula disclosed in each of the Patent Documents 1 and 2 is disadvantageous in the following respects. If the cannula has a small inner diameter, a load is imposed upon operator's fingers when the syringe engaged with the cannula is pressed by the fingers to jet the liquid in the cannula through the nozzle orifices. Therefore, when the liquid is applied with pressure to be jetted, the operator's fingers may be undesiredly moved. In this event, the liquid can not accurately be directed to a predetermined jet position at a treated part. As a consequence, the treated part can not properly and quickly be treated.

In case where the forward end portion of the cannula is directed downward, the liquid may undesirably flow out through the nozzle orifices of the cannula forward and downward from the forward end portion under no pressure, i.e., before and after the liquid is jetted under pressure. Such phenomenon may be called dripping. Before the liquid is jetted under pressure, the liquid is filled in the cannula and is not yet applied with pressure. After the liquid is jetted under pressure, the pressure is released to zero. In the event that the dripping occurs during the dental treatment before and after the liquid is jetted under pressure, the liquid may flow into another area of an oral cavity other than the treated part or flow out from the oral cavity.

In case where the liquid is applied with low pressure, the liquid is jetted forward from the cannula in a small angle range. In this event, it is impossible to jet the liquid to an entire area of a gap between the tooth and the gingiva and to sufficiently supply the liquid over lateral surfaces of the tooth and the gingiva opposite to each other.

For example, the cannula disclosed in each of the Patent Documents 1 and 2 is used in a state where the forward end portion of the cannula is plunged and inserted into the periodontal pocket as the treated part. In this event, the treated part may possibly be damaged by excessive plunging. Thus, the safety of a patient can not be assured.

Further, the cannula in each of the Patent Documents 1 and 2 is not adapted to cleaning of a dental root furcation present in a multi-rooted tooth having two or more dental roots and cleaning of a periodontal pocket present at the dental root furcation.

The cannula disclosed in each of the Patent Documents 1 and 2 is disadvantageous in that the processing efficiency is low because a plurality of nozzle orifices are formed one by one in the single cannula.

Further, the cannula disclosed in each of the Patent Documents 1 and 2 is disadvantageous in that, if the nozzle orifices are formed by the use of a grindstone, burrs are produced around an edge of each orifice. Therefore, an additional step of removing the burrs is required.

In the cannula disclosed in Patent Document 2, the forward end portion of the cannula is simply bent in one direction or simply shaped into a wide or gentle curve. Therefore, in case where the treatment is performed in a narrow oral cavity in the dental field, it is difficult to treat the treated part on the left, the right, the top, or the bottom in the oral cavity by directing the cannula to a desired position.

Further, the cannula disclosed in the Patent Document 2 has flexibility and is bent by the operator's hand in conformity with the treated part. In this event, the shape of the cannula may be changed during the treatment so that precise and proper treatment is difficult.

It is therefore an object of this invention to provide a liquid injection needle element which is capable of accurately and efficiently jetting a liquid in a predetermined angle range under low pressure.

It is another object of this invention to provide a liquid injection needle element which is capable of preventing dripping, i.e., a phenomenon that a liquid undesiredly flows out from a cannula through a nozzle orifice under no pressure before and after the liquid is jetted under pressure.

It is still another object of this invention to provide a liquid injection needle element which is capable of further smoothly jetting a liquid in a predetermined direction.

It is yet another object of this invention to provide a liquid injection needle element which is prevented from damaging a living tissue.

It is another object of this invention to provide a liquid injection needle element which is capable of assuring safety and convenience in use.

It is another object of this invention to provide a method of producing a liquid injection needle element, which is capable of easily forming a nozzle orifice in a cannula at its front part.

It is another object of this invention to provide a method of producing a liquid injection needle element, which is capable of efficiently forming a nozzle orifice at a front part of a cannula without occurrence of burrs and which is suitable for mass-production.

According to this invention, there is provided a liquid injection needle element comprising a cannula having a front part in a direction of a center axis with its forward end portion closed, the cannula having a plurality of nozzle orifices formed on the front part and spaced from one another so as to allow a liquid in the cannula to be jetted out of the cannula when the liquid is applied with pressure, wherein each of the nozzle orifices has an orifice axis intersecting the center axis, each of the nozzle orifices having a first diameter in a first direction and a second diameter in a second direction intersecting the first direction, the first diameter ranging between 0.1 mm and 0.5 mm, the second diameter ranging between 0.1 mm and 0.3 mm, the liquid being applied with pressure ranging between 19.613 x 10³ Pa and 196.133 x 10³ Pa to jet the liquid from the nozzle orifices in an angle range of 45 degrees towards the forward end portion with respect to the orifice axis.

Preferably, the nozzle orifices are formed at positions shifted in angle from one another around the center axis.

Preferably, the cannula is provided with a plurality of index marks formed on its outer surface at predetermined positions from the forward end portion in the direction of the center axis.

Preferably, each of the nozzle orifices is surrounded by a marginal wall surface coated with a resin layer.

According to this invention, there is provided a liquid injection needle element comprising a cannula having a front part in a direction of a center axis with its forward end portion closed, the cannula having a plurality of nozzle orifices formed on the front part and spaced from one another so as to allow a liquid in the cannula to be jetted out of the cannula when the liquid is applied with pressure, wherein the nozzle orifices are spaced from one another in the direction of the center axis, the cannula having a base extending along the center axis and a curved portion extending from one end of the base towards the front part of the cannula and bent around the center axis.

Preferably, each of the nozzle orifices has an orifice axis intersecting the center axis. Each of the nozzle orifices has a first diameter in a first direction and a second diameter in a second direction intersecting the first direction. The first diameter ranges between 0.1 mm and 0.5 mm. The second diameter ranges between 0.1 mm and 0.3 mm. The liquid is applied with pressure ranging between 19.613 x 10³ Pa and 196.133 x 10³ Pa to jet the liquid from the nozzle orifices in an angle range of 45 degrees towards the forward end portion with respect to the orifice axis.

Preferably, the nozzle orifices are formed at positions shifted in angle from one another around the center axis.

Preferably, the cannula is provided with a plurality of index marks formed on its outer surface at predetermined positions from the forward end portion in the direction of the center axis.

Preferably, each of the nozzle orifices is surrounded by a marginal wall surface coated with a resin layer.

According to this invention, there is provided a method of producing a liquid injection needle element comprising a cannula having a front part in a direction of a center axis with its forward end portion closed, the cannula having a plurality of nozzle orifices formed on the front part and spaced from one another so as to allow a liquid in the cannula to be jetted out of the cannula when the liquid is applied with pressure, the method comprising the steps of preparing a tubular workpiece, facing a discharge wire to an outer surface of the workpiece in a direction intersecting the center axis of the workpiece, and moving the discharge wire to form the nozzle orifice by wire electric discharge, moving the discharge wire in the direction of the center axis to successively form the nozzle orifices at a front part of the workpiece with a space kept from one another in the direction of the center axis, and sealing and closing a forward end portion of the workpiece in the direction of the center axis.

The method may further comprise the step of bending a portion of the workpiece between the front part and a base opposite to the front part around the center axis of the workpiece to form a curved portion.

Preferably, each of the nozzle orifices has an orifice axis intersecting the center axis. Each of the nozzle orifices has a first diameter in a first direction and a second diameter in a second direction intersecting the first direction. The first diameter ranges between 0.1 mm and 0.5 mm. The second diameter ranges between 0.1 mm and 0.3 mm. The liquid is applied with pressure ranging between 19.613 x 10³ Pa and 196.133 x 10³ Pa to jet the liquid from the nozzle orifices in an angle range of 45 degrees towards the forward end portion with respect to the orifice axis.

Preferably, one of the discharge wire and the workpiece is moved in the direction of the center axis after each nozzle orifice is formed. The workpiece is rotated around the center axis by a predetermined angle so that the nozzle orifices are formed at positions angularly shifted from one another around the center axis.

Preferably, the cannula is provided with a plurality of index marks formed on its outer surface at predetermined positions from the forward end portion in the direction of the center axis.

Preferably, each of the nozzle orifices is surrounded by a marginal wall surface coated with a resin layer.

Preferably, in the sealing step, an outer surface of the forward end portion of the cannula is formed into a semispherical shape by laser machining to close the forward end portion.

Now, several preferred embodiments of this invention will be described with reference to the drawing, in which:
Fig. 1 is a front view of a liquid injection needle element according to a first embodiment of this invention with a needle hub shown in section;
Fig. 2 is an enlarged front view of a front part of a cannula illustrated in Fig. 1;
Fig. 3 is a right side view of the cannula illustrated in Fig. 2;
Fig. 4 is a sectional view taken along a line IV-IV in Fig. 2;
Fig. 5 is an enlarged front view of a front part of a cannula of a liquid injection needle element according to a second embodiment of this invention;
Fig. 6 is a sectional view taken along a line VI-VI in Fig. 5;
Fig. 7 is an enlarged front view of a front part of a cannula of a liquid injection needle element according to a third embodiment of this invention;
Fig. 8 is a right side view of the cannula illustrated in Fig. 7;
Fig. 9 is an enlarged front view of a front part of a cannula of a liquid injection needle element according to a fourth embodiment of this invention;
Fig. 10 is a right side view of the cannula illustrated in Fig. 9;
Fig. 11A is a front view of a liquid injection needle element as a first specific example according to a fifth embodiment of this invention;
Fig. 11B is a right side view of a cannula illustrated in Fig. 11A;
Fig. 12A is a front view of a liquid injection needle element as a second specific example according to the fifth embodiment of this invention;
Fig. 12B is a right side view of a cannula illustrated in Fig. 12A;
Fig. 13A is a front view of a liquid injection needle element as a third specific example according to the fifth embodiment of this invention;
Fig. 13B is a right side view of a cannula illustrated in Fig. 13A;
Fig. 14 is a view for describing a wire electric discharge machine used in a method of producing a liquid injection needle component according to this invention;
Fig. 15 is a view for describing a laser machining apparatus used in the method; and
Fig. 16 is a view for describing a step of processing a forward end portion of a cannula using the laser machining apparatus.

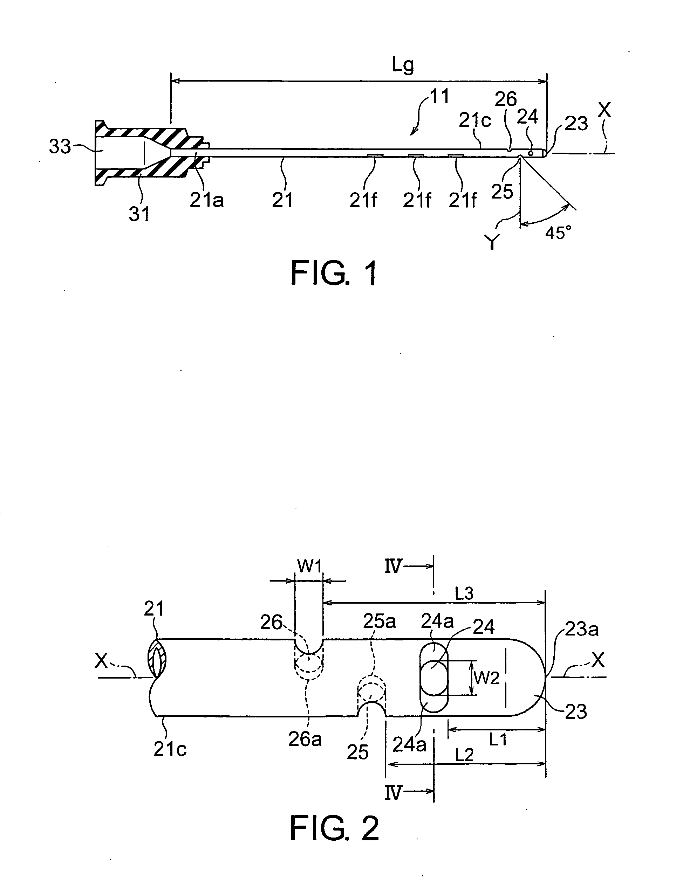

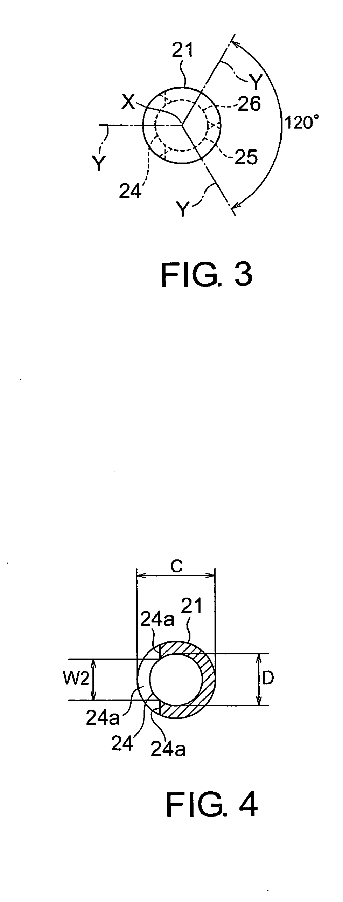

Referring to Figs. 1 through 4, a liquid injection needle element 11 according to a first embodiment of this invention comprises a cylindrical cannula 21 and a needle hub 31 holding the cannula 21.

The cannula 21 is made of a metal material and has a base 21 a and a front part 21c opposite to the base 21a. The base 21a is supported by the needle hub 31. The front part 21 c has a forward end portion 23 as a closed end. The forward end portion 23 has a semispherical outer surface. The front part 21 c of the cannula 21 is provided with a first nozzle orifice 24, a second nozzle orifice 25, and a third nozzle orifice 26 formed on a lateral wall thereof.

The first through the third nozzle orifices 24, 25, and 26 are spaced from one another in a direction of a center axis X of the cannula 21. Each of the first through the third nozzle orifices 24, 25, and 26 has an orifice axis Y perpendicular to the center axis X. The first through the third nozzle orifices 24, 25, and 26 are also spaced from one another in a circumferential direction around the center axis X so that the orifice axes Y are spaced by 120 degrees from one another. Thus, the first through the third nozzle orifices 24, 25, and 26 are located at different positions on the lateral wall of the front part 21c of the cannula 21, i.e., at positions different both in the circumferential direction and in the direction of the center axis X.

The first through the third nozzle orifices 24, 25, and 26 are formed inside marginal wall surfaces 24a, 25a, and 26a depressed from an outer peripheral surface of the cannula 21 towards the center axis X. Each of the marginal wall surfaces 24a, 25a, and 26a has a generally U-shaped section.

The needle hub 31 is made of a resin material. The needle hub 31 has a coupling portion 33 to be coupled with an end of a syringe tube of a syringe (not shown). A liquid contained in the syringe tube is introduced into the cannula 21 from the end of the syringe tube under low pressure and is jetted from the first through the third nozzle orifices 24, 25, and 26 to the outside of the cannula 21.

The cannula 21 mentioned above has an overall length Lg in the direction of the center axis X, an outer diameter C, and an inner diameter D. These dimensions of the cannula 21 as well as dimensions and positions of the first through the third nozzle orifices 24, 25, and 26 are selected within predetermined ranges.

It is important to jet the liquid from each of the first through the third nozzle orifices 24, 25, and 26 in an angle range of 45 degrees towards the forward end portion 23 with respect to the orifice axis Y. Specifically, by jetting the liquid within the angle range of 45 degrees under low pressure, the liquid can be delivered to an entire lateral surface of a periodontal pocket formed between a tooth and a gingiva. Simultaneously, the liquid can flow to the bottom of the periodontal pocket. Thus, the above-mentioned angle range is ideal for the periodontal pocket.

In the cannula 21, consideration must be made about the change in jetting pressure depending upon the jetting amount of the liquid in each of the first through the third nozzle orifices 24, 25, and 26. Further, before and after the liquid is jetted under pressure, natural dripping of the liquid out of the cannula 21 must be prevented.

As mentioned above, the dripping is a phenomenon that the liquid drips down through the first through the third nozzle orifices 24, 25, and 26 under no pressure before and after the liquid is jetted under pressure. Before the liquid is jetted under pressure, the liquid is filled in the cannula 21 and is not yet applied with pressure. After the liquid is jetted under pressure, the pressure is released to zero.

Each of the first through the third nozzle orifices 24, 25, and 26 has a first diameter W1 in a first direction parallel to the center axis X and a second diameter W2 in a second direction perpendicular to the first direction.

Each of the first through the third nozzle orifices 24, 25, and 26 has an arc shape on opposite sides in the circumferential direction perpendicular to the center axis X.

Hereinafter, a specific example of the cannula 21 will be described. The cannula 21 has the overall length Lg of 32 mm, the outer diameter C of 0.6 mm, and the inner diameter D of 0.3 mm. Each of the first through the third nozzle orifices 24, 25, and 26 has the first diameter W1 of 0.22 mm and the second diameter W2 of 0.2 mm.

Referring to Fig. 2, the first through the third nozzle orifices 24, 25, and 26 are formed at distances L1, L2, and L3 from an end point 23a of the forward end portion 23 of the cannula 21 in the direction of the center axis X, respectively. In detail, each of the distances L1, L2, and L3 is measured as a distance between the end point 23a and an edge of each of the first through the third nozzle orifices 24, 25, and 26 which is nearest to the end point 23a. Herein, L1, L2, and L3 are equal to 1.00 mm, 1.3 mm, and 1.60 mm, respectively.

The liquid in the cannula 21 is applied with pressure of 24.516 x 10³ Pa (= 250 gf/cm²). It has been revealed that, under the above-mentioned pressure, the liquid is jetted from the first through the third nozzle orifices 24, 25, and 26 in the angle range of 45 degrees.

When the liquid is applied with pressure, the amount of liquid jetted from the first nozzle orifice 24 is 33% with respect to the total amount jetted from all of the first through the third nozzle orifices 24, 25, and 26. The amount of liquid jetted from the second and the third nozzle orifices 25 and 26 are 37% and 30%, respectively.

If the inner diameter D of the cannula 21 is as small as between 0.1 mm and 0.5 mm, it is most desirable in view of the operability that the liquid is jetted from the first through the third nozzle orifices 24, 25, and 26 in the angle range of 45 degrees under low pressure within a range between 19.613 x 10³ Pa and 196.133 x 10³ Pa (between 200 gf/cm² and 2 kgf/cm²). Thus, in order to jet the liquid in the angle range of 45 degrees, each of the first through the third nozzle orifices 24, 25, and 26 is required to have the first diameter W1 between 0.1 mm and 0.5 mm and the second diameter W2 between 0.1 mm and 0.3 mm.

As described above, depending upon the inner diameter D of the cannula 21 and the pressure applied to the liquid, the first and the second diameter W1 and W2 of the first through the third nozzle orifices 24, 25, and 26 are appropriately selected so as to jet the liquid within the angle range of 45 degrees.

Each of the first through the third nozzle orifices 24, 25, and 26 may have a polygonal shape such as a long rectangular shape or a round shape such as an elliptical shape.

Each of the first through the third nozzle orifices 24, 25, and 26 may be arranged so that the first direction and the second direction perpendicular to the first direction are inclined with respect to the center axis X. In any event, the first and the second diameters W1 and W2 of the first through the third nozzle orifices 24, 25, and 26 must satisfy predetermined ranges.

The cannula 21 is provided with a polytetrafluoroethylene resin layer as a water repellent layer formed on the marginal wall surfaces 24a, 25a, and 26a around the first through the third nozzle orifices 24, 25, and 26. In this case, the liquid is applied with pressure of 19.613 x 10³ Pa (200 gf/cm²) so as to jet the liquid from the first through the third nozzle orifices 24, 25, and 26 in the angle range of 45 degrees.

Thus, in case where the resin layer is formed on the marginal wall surfaces 24a, 25a, and 26a, the resin layer serves to reduce frictional resistance between the liquid and the marginal wall surfaces. Therefore, the pressure required to jet the liquid can further be lowered.

As illustrated in Fig. 1, the cannula 21 is provided with a plurality of index marks 21f formed on its outer surface at predetermined positions between the forward end portion 23 of the front part 21 c and the needle hub 31 in the direction of the center axis X. Specifically, the index marks 21f are formed at positions of 3 mm, 5 mm, and 7 mm from the forward end portion 23 of the cannula 21 in the direction of the center axis X.

In advance, the depth of an affected part (for example, a periodontal pocket) of a patient is measured by a dental probe. Then, when the forward end portion 23 of the cannula 21 is inserted into the affected part, an insertion depth is monitored by the use of the index marks 21f with reference to the depth of the affected part as measured. Thus, the index marks 21f serve as a guide when the forward end portion 23 of the cannula 21 is inserted.

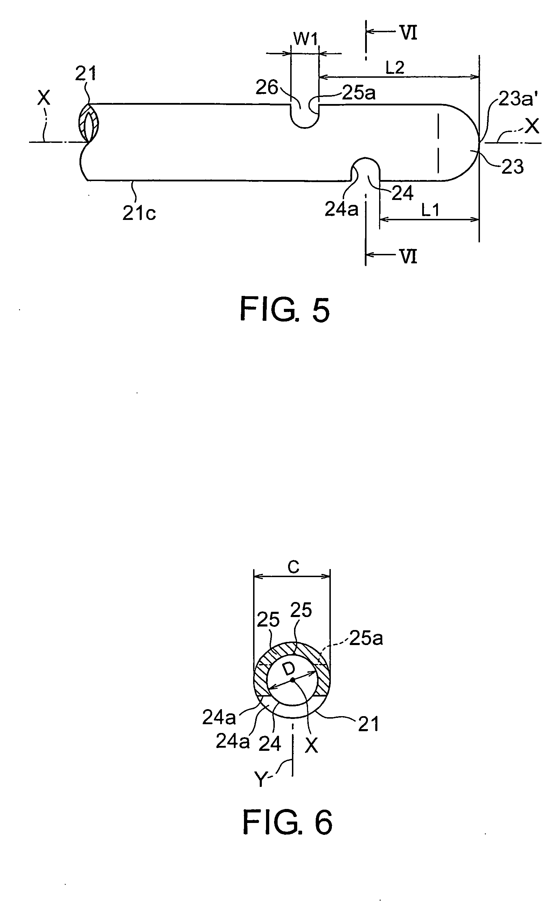

Referring to Figs. 5 and 6, description will be made of a liquid injection needle element according to a second embodiment of this invention. Similar parts are designated by like reference numerals and description thereof will be omitted.

As illustrated in the figures, the cannula 21 has only the first and the second nozzle orifices 24 and 25 without the third nozzle orifice 26. The first and the second nozzle orifices 24 and 25 are formed on the lateral wall of the cannula 21 at its front part and at positions different from each other in the direction of the center axis X of the cannula 21. The first and the second nozzle orifices 24 and 25 are spaced from each other in the circumferential direction of the lateral wall around the center axis X so that the orifice axes Y are spaced by 180 degrees from each other.

In the cannula 21 in the second embodiment, when the forward end portion 23 of the cannula 21 is directed downward, the liquid can be jetted from each of the first and the second nozzle orifices 24 and 25 in the angle range of 45 degrees with respect to the orifice axis Y

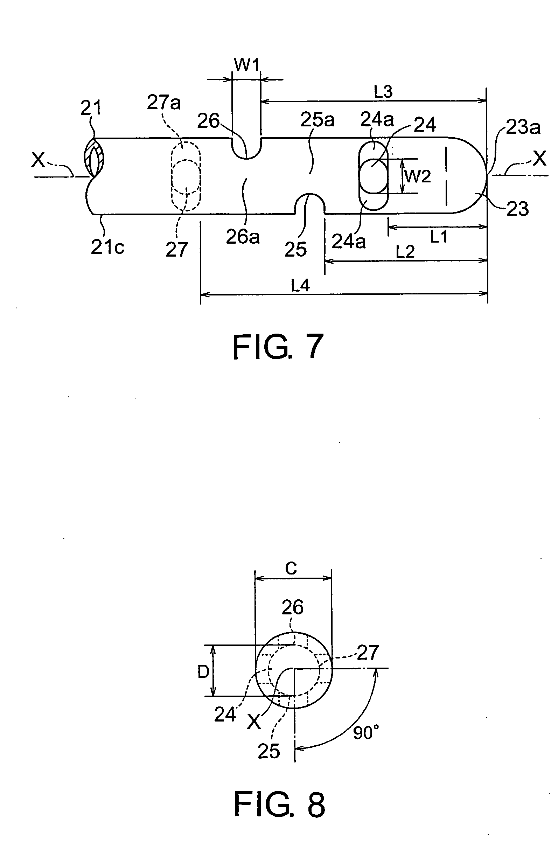

Referring to Figs. 7 and 8, description will be made of a liquid injection needle element according to a third embodiment of this invention. Similar parts are designated by like reference numerals and description thereof will be omitted.

The cannula 21 has a fourth nozzle orifice 27 in addition to the first through the third nozzle orifices 24, 25, and 26 in the first embodiment. The first through the fourth nozzle orifices 24 through 27 are formed on the lateral wall of the cannula 21 at its front part 21 c and at positions different from one another in the direction of the center axis X of the cannula 21. The first through the fourth nozzle orifices 24 through 27 are spaced from each other in the circumferential direction around the center axis X so that the orifice axes Y are spaced by 90 degrees from one another.

The fourth nozzle orifice 27 is same in shape as the first through the third nozzle orifices 24, 25, and 26. The fourth nozzle orifice 27 is formed at a distance L4 of 1.90 mm as a distance in the direction of the center axis X between the end point 23a of the cannula 21 and an edge of the fourth nozzle orifice 27 which is nearest to the end point 23a.

In the cannula 21 in the third embodiment, when the forward end portion 23 of the cannula 21 is directed downward, the liquid can be jetted from each of the first through the fourth nozzle orifices 24 through 27 in the angle range of 45 degrees with respect to the orifice axis Y.

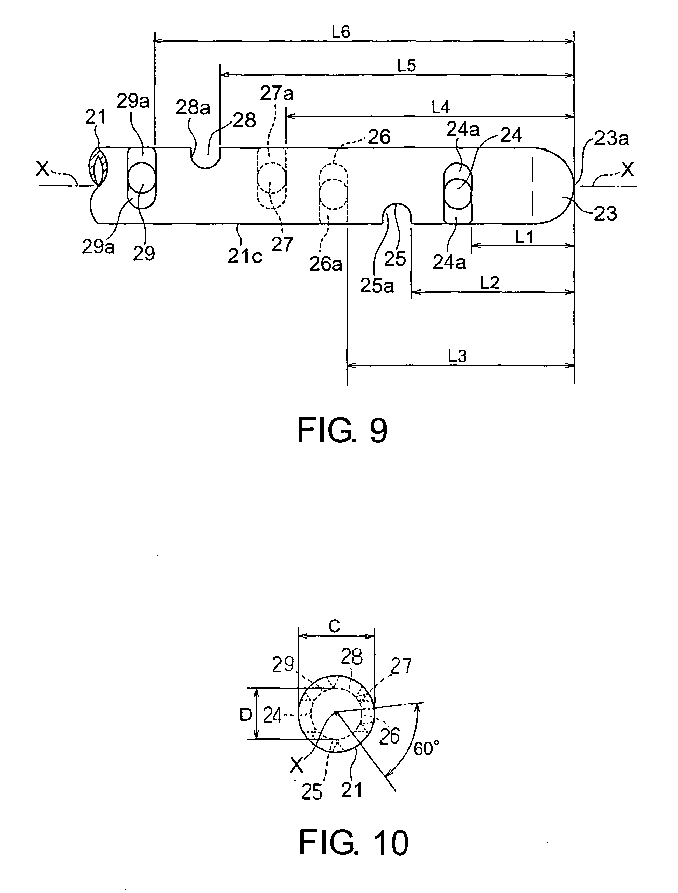

Referring to Figs. 9 and 10, description will be made of a liquid injection needle element according to a fourth embodiment of this invention. Similar parts are designated by like reference numerals and description thereof will be omitted.

As shown in Figs. 9 and 10, the cannula 21 in the fourth embodiment has fifth and sixth nozzle orifices 28 and 29 in addition to the first through the fourth nozzle orifices 24 through 27 in the third embodiment.

The first through the sixth nozzle orifices 24 through 29 are formed on the lateral wall of the cannula 21 at its front part 21 c and at positions different from one another in the direction of the center axis X of the cannula 21. The first through the sixth nozzle orifices 24 through 29 are spaced from one another in the circumferential direction around the center axis X so that the orifice axes Y are spaced by 60 degrees from one another.

The fifth nozzle orifice 28 is same in shape as the first through the fourth nozzle orifices 24, 25, 26, and 27. The fifth nozzle orifice 28 is formed at a distance L5 of 2.20 mm as a distance in the direction of the center axis X between the end point 23a of the cannula 21 and an edge of the fifth nozzle orifice 28 which is nearest to the end point 23a.

The sixth nozzle orifice 29 is same in shape as the first through the fifth nozzle orifices 24 through 28. The sixth nozzle orifice 29 is formed at a distance L6 of 2.50 mm as a distance in the direction of the center axis X between the end point 23a of the cannula 21 and an edge of the sixth nozzle orifice 29 which is nearest to the end point 23a.

In the cannula 21 in the fourth embodiment, when the forward end portion 23 of the cannula 21 is directed downward, the liquid can be jetted from each of the first through the sixth nozzle orifices 24 through 29 in the angle range of 45 degrees with respect to the orifice axis Y

In case where the liquid described in the first through the fourth embodiments is a cleaning liquid, 10% sodium hypochlorite solution, 3% hydrogen peroxide solution, or the like may be used. The number of the nozzle orifices are not restricted to the numbers specified in the first through the fourth embodiments but may be any appropriate number as far as the nozzle orifices are formed at two to six different positions in the direction of the center axis X.

In the foregoing embodiments, the nozzle orifices are arranged in the circumferential direction of the cannula 21 at equal angles from one another. Alternatively, the nozzle orifices may be arranged at different angles.

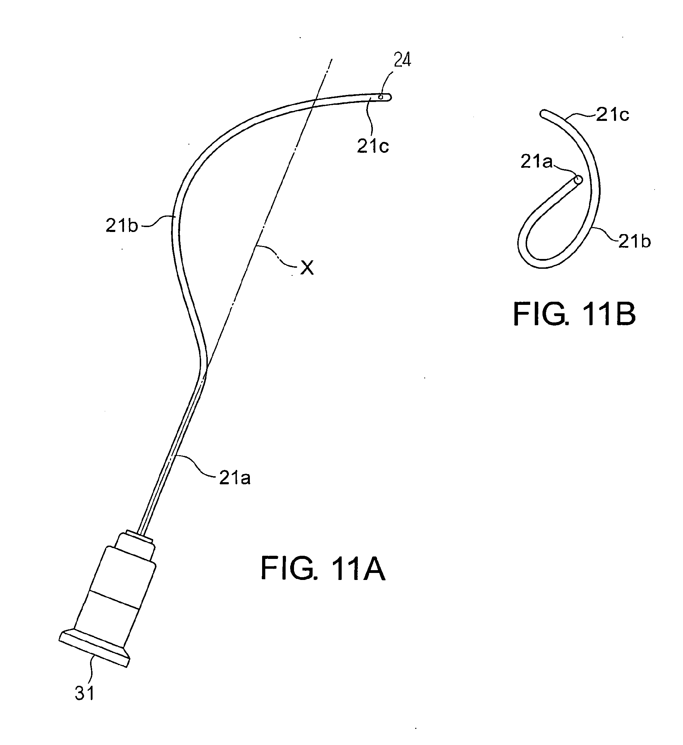

Referring to Figs. 11A and 11 B, a liquid injection needle element as a first specific example of a fifth embodiment of this invention is a modification of each of the first through the fourth embodiments. Specifically, the cannula 21 in the fifth embodiment is bent into a special shape. The cannula 21 described in each of the first through the fourth embodiments comprises a stainless-steel tube. However, the cannula 21 may easily be produced by the use of other metal tubes, alloy tubes, resin tubes, and so on.

The first through the sixth nozzle orifices 24 through 29 described in the first through the fourth embodiments may be formed by boring or drilling the lateral wall of the cannula 21 at its front part 21 c.

The cannula 21 illustrated in Figs. 11 A and 11 B has the base 21 a extending from the needle hub 31 (Fig. 1) in the direction of the center axis X, a curved portion 21 b extending from one end of the base 21 a towards the forward end portion 23 of the cannula 21 and bent away from the center axis X and around the center axis X with a space kept therefrom, and the front part 21 c extending from one end of the curved portion 21 b away from the center axis X of the base portion 21 a.

As shown in Fig. 11B, the curved portion 21 b is bent rightward around the center axis X and then deeply bent leftward. Thus, the curved portion 21 b is bent to form a part of a spiral from the base 21 a to the front part 21 c. The front part 21 c extends from the one end of the curved portion 21 b towards the forward end portion 23 forward from the curved portion 21 b.

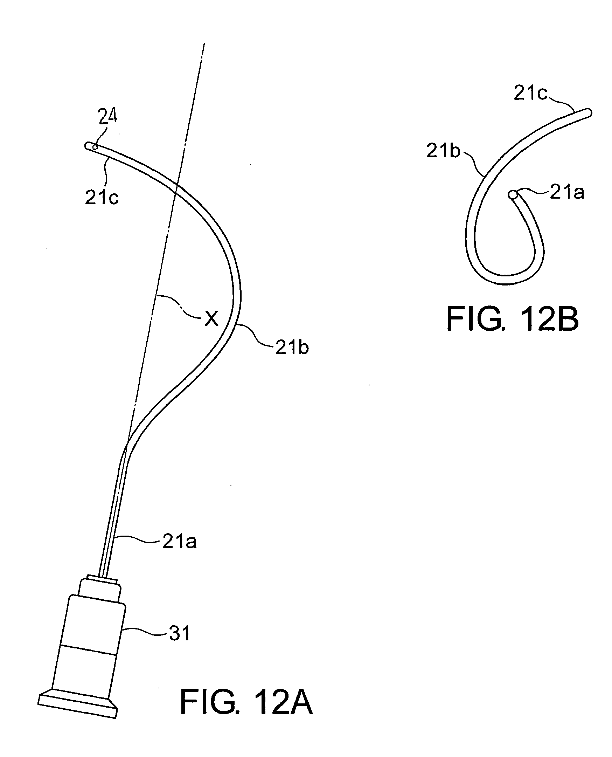

Figs. 12A and 12B show a modification of the cannula 21 as a second specific example of the fifth embodiment. As shown in Fig. 12B, the cannula 21 has the curved portion 21 b bent leftward around the center axis X and then deeply bent rightward. Thus, the curved portion 21 b is bent to form a part of a spiral from the base 21 a to the front part 21 c. The front part 21 c extends from the one end of the curved portion 21 b towards the forward end portion 23 forward from the curved portion 21 b in the direction of the center axis X.

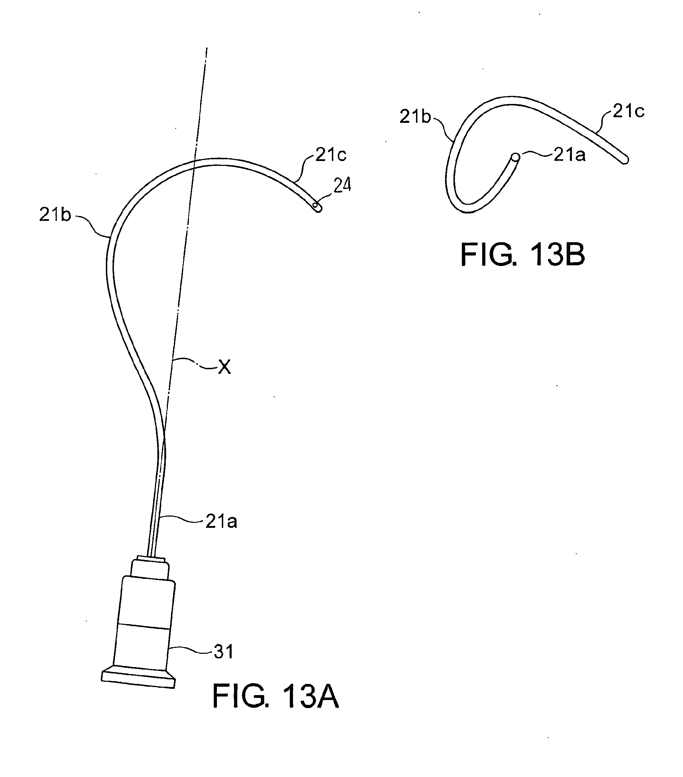

Figs. 13A and 13B show a modification of the cannula 21 as a second specific example of the fifth embodiment. As shown in Fig. 13B, the cannula 21 has the curved portion 21 b bent leftward around the center axis X and then deeply bent leftward. Thus, the curved portion 21 b is bent to form a part of a spiral from the base 21 a to the front part 21 c. The front part 21 c extends from the one end of the curved portion 21 b towards the forward end portion 23 rearward from the curved portion 21 b in the direction of the center axis X.

The front part 21c of each of the cannulas 21 illustrated in Figs. 11A through 13B is continuously bent along a curve of the curved portion 21 b. Alternatively, the front part 21 c may be straight without being bent.

In the fifth embodiment, the cannulas 21 have various shapes illustrated in Figs. 11 A through 13B. For example, when an affected part in the oral cavity is treated, the position of the affected part is at first identified. Depending upon the position of the affected part, the cannulas 21 are selectively used in the treatment. The front part 21 c of each cannula 21 of the fifth embodiment is provided with any of the first through the sixth nozzle orifices 24 through 29 described in conjunction with the first through the fourth embodiments.

Each of the cannulas 21 of the liquid injection needle element illustrated in Figs. 11A through 13B is has a shape suitable for treatment of upper and lower teeth in the depth of the oral cavity. In particular, the cannula 21 is suitable for cleaning the inside of a dental root furcation in a first molar tooth among the teeth located in the depth of the oral cavity.

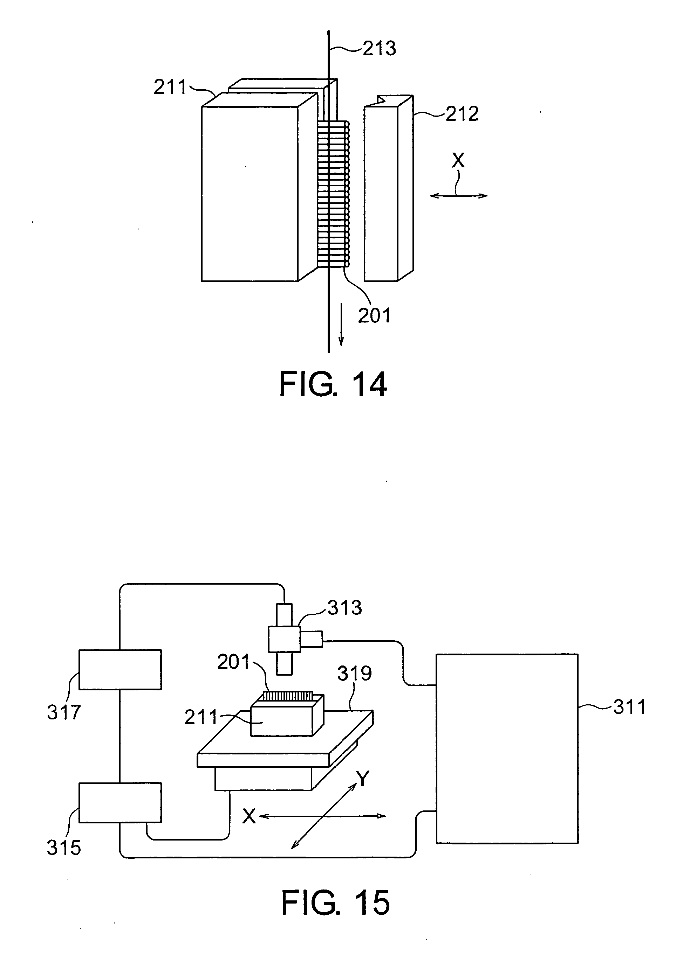

Now, referring to Figs. 14 to 16, description will be made of a method of producing a liquid injection needle element in each of the first through the fifth embodiments.

Referring to Fig. 14, a wire electric discharge machine comprises a pair of fixing tools 211 for holding a workpiece 201 as an unprocessed cylindrical metal tube except its front part, a moving tool 212, and a discharge wire 213.

In wire electric discharge, a plurality of workpieces 201 are arranged in a flat plane and held by the fixing tools 211 so that the nozzle orifices of the workpieces 201 can be simultaneously formed. Each workpiece 201 is held by the fixing tools 211 except the front part. A minimum length of the front part is protruded from the fixing tools 211. The fixing tools 211 are designed to be used in common to various steps so that, once the workpieces 201 are set, the workpieces 201 are continuously held by the fixing tools 211 until completion of a production process.

Referring to Fig. 15, a laser machining apparatus for use in machining the liquid injection needle element comprises a laser generating portion 311, a laser irradiating portion 313, a NC (numerical control) portion 315, an image processing portion 317, and a set tool 319 for setting the workpieces 201.

The cannula 21 described in each of the first through the fourth embodiments is produced via a boring step of forming a plurality of nozzle orifices by subjecting the workpiece 201 to wire electric discharge using the wire electric discharge machine, a cleaning step of cleaning the workpiece 201 using a ultrasonic cleaner, and a sealing step of sealing a forward end portion of the workpiece 201 by laser irradiation using the laser machining apparatus.

Hereinafter, the production process of the cannula 21 of the liquid injection needle element described in the first embodiment will be described by way of example.

At first, a plurality of workpieces 201 are prepared. An outer surface of the discharge wire 213 is faced to outer surfaces of the workpieces 201 and extends in a direction perpendicular to the center axes X of the workpieces 201 and the orifice axes Y. The discharge wire 213 is moved to melt desired positions of the workpieces 201. Thus, the first nozzle orifices 24 are formed by wire electric discharge.

Alternatively, the outer surface of the discharge wire 213 may be faced to the outer surfaces of the workpieces 201 in a direction intersecting the center axes X of the workpieces 201 and the orifice axes Y. Then, the wire electric discharge is performed by moving the discharge wire 213 to melt desired positions of the workpieces 201. Thus, the first nozzle orifices 24 are formed.

Thereafter, the discharge wire 213 is moved in the direction of the center axis X and the wire electric discharge is carried out to form the second nozzle orifices 25 at positions spaced from the first nozzle orifices 24 in the direction of the center axis X. Finally, the discharge wire 213 is moved in the direction of the center axis X and the wire electric discharge is carried out to form the third nozzle orifices 26 at positions spaced from the second nozzle orifices 25 in the direction of the center axis X.

When the workpiece 201 is drilled to form the first through the third nozzle orifices 24, 25, and 26, one of the discharge wire 213 and the workpiece 201 is moved in the direction of the center axis X. Further, when the first through the third nozzle orifices 24, 25, and 26 are formed, the workpiece 201 is rotated around the center axis X by predetermined angles so that the first through the third nozzle orifices 24, 25, and 26 are formed at positions angularly shifted around the center axis X.

The discharge wire 213 has an outer diameter of 0.5 mm or less. For example, if the discharge wire 213 has an outer diameter of about 0.2 mm, the nozzle orifice having the first diameter W1 of 0.5 mm in the direction of the center axis X is formed by slightly shifting the discharge wire 213 in the direction of the center axis X.

The position, the shape, and the size of the nozzle orifice formed in the workpiece 201 may freely be changed by a NC (numerical control) program.

In order to form the curved portion 21 b as in the fifth embodiment described in conjunction with Figs. 11A through 13B, a portion of the workpiece 201 between the front part 21 c and the base 21 a opposite to the front part 21 c is bent around the center axis X of the workpiece 201.

Further, the marginal wall surfaces 24a, 25a, and 26a formed around the first through the third nozzle orifices 24, 25, and 26 of the cannula 21 may be coated with a resin layer if desired.

In the wire electric discharge, a large number of (100 to 200) workpieces can be processed in a single cycle. By the use of the fixing tool and the setting tool for fixing and setting the workpieces and the NC portion, high-precision working is possible. Thus, mass-production with a stable quality is achieved.

In the wire electric discharge, no burrs are produced so that post-processing is easy. In order to round an edge of the nozzle orifice formed by the wire electric discharge, polishing by a magnetic barrel may be carried out so as to improve external appearance and product performance such as safety and reliability. The polishing using the magnetic barrel does not require chemicals used in chemical polishing.

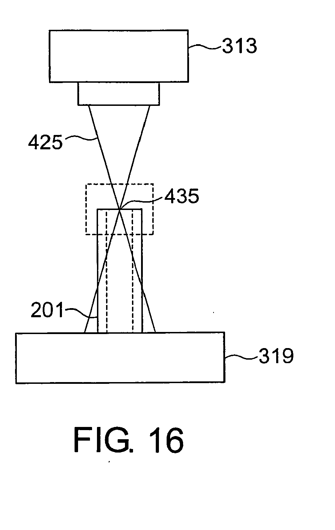

Referring to Fig. 16, the sealing step will be described. In the sealing step, forward end processing is carried out. Specifically, an outer surface of a forward end portion of the workpiece 201 is processed into a semispherical shape. In the sealing step, output conditions of the laser irradiating portion 313 are determined depending upon the diameter of the workpiece 201. Laser light 425 is irradiated so that a focal point 435 is coincident with the center of the forward end portion of the workpiece 201. At this time, automatic centering is performed by the use of the image processing portion 317 and the NC portion 315.

The workpieces 201 are arranged at a predetermined pitch to be continuously processed at a high processing speed. As the laser light, a YAG (pulse) laser is used. Not one pulse but several pulses of the laser light are irradiated in a predetermined time period. The focal distance, the focal position and the focal depth are determined so that the laser light has a beam diameter greater by 0.1 mm than the diameter of the workpiece 201.

Thus, by appropriately determining the time period of laser irradiation and the number of pulses, the forward end portion of the workpiece 201 is gradually melted to form the semispherical portion.

Further, the semispherical portion can be efficiently formed at a high speed, i.e., by laser irradiation of about 7/100 sec. Therefore, high-quality and low-price products can be obtained. Specifically, 100 workpieces 201 are set and continuously processed at a rate of 1 second per each single workpiece. During processing, the laser light is irradiated with an inexpensive nitrogen gas used for preventing oxidization.

The sealing step may be performed prior to formation of the first through the third nozzle orifices 24, 25, and 26.

In the foregoing, the method of producing the cannula according to this invention has been described in connection with the first embodiment. However, the cannula in each of the second through the fifth embodiments can also be produced in the similar manner.

The liquid injection needle element according to this invention is capable of accurately and efficiently jetting the liquid to a desired position in the angle range of 45 degrees under low pressure.

When the liquid is applied with no pressure, it is possible to prevent the dripping that the liquid flows out from the cannula through the nozzle orifices.

If the marginal wall surface around the nozzle orifice of the cannula is coated with the resin layer, friction between the liquid and the marginal wall surface is reduced. Therefore, the liquid can be more smoothly jetted in a predetermined direction.

In the liquid injection needle element, the portion of the cannula between the front part and the base may be curved at a desired angle in a desired direction with respect to the base. In addition, the front part may be similarly curved. With this structure, the cannula can easily be inserted into a curved root canal or into a periodontal pocket in a dental root furcation in the dental field. Thus, the liquid injection needle element is very convenient in use.

When the treatment is performed in a narrow oral cavity, the cannula having the shape suitable for a treated part on the left, the right, the top, or the bottom in the oral cavity is appropriately selected and used. Thus, the operation efficiency is improved.

The outer surface of the forward end portion of the cannula has a semispherical shape formed by laser machining. It is therefore possible to prevent a living tissue from being damaged when the cannula is inserted to the affected part to jet the liquid.

By providing the cannula having the nozzle orifices with a plurality of index marks formed at its front part, an insertion depth of the cannula is monitored by the use of the index marks with reference to the depth of affected part as preliminarily measured by the dental probe. Therefore, the treatment is carried out without applying excessive pressure to the bottom of the periodontal pocket.

Thus, in the treatment of the periodontal pocket, it is possible to assure more safety and convenience in use.

Further, according to the method of this invention, it is possible to easily form the nozzle orifices at the front part of the cannula by wire electric discharge. By the wire electric discharge, the nozzle orifices can be efficiently formed without occurrence of burrs and can simultaneously be formed in a number of cannulas. Thus, the method of this invention is suitable for mass-production.

The liquid injection needle element is also applicable to root canal cleaning for removing dentin chips or residual organic substances during formation of a root canal in the dental field and to injection of anesthetic solution or hemostatic solution to the affected part.

Further, the liquid injection needle element is applicable to injection of a chemical solution for preventing avian influenza or bird flu into an egg immediately before hatching. When the chemical solution is injected into the egg, it is necessary to plunge the forward end portion of the cannula from the outside to the inside of an eggshell via a hole of a minimum diameter so that a bird immediately before hatching is not damaged and that the eggshell is not broken.

Specifically, the forward end portion of the cannula 21 provided with the first through the third nozzle orifices 24, 25, and 26 illustrated in Fig. 1 is inserted to the inside of the eggshell. Inside the eggshell, a predetermined amount of the chemical solution effective to bird flu virus is jetted from the first through the third nozzle orifices 24, 25, and 26. At this time, since the forward end portion of the cannula 21 has a semispherical surface, the chemical solution can be injected along an inner surface of the eggshell without damaging the bird in the eggshell. Thus, the liquid injection needle element is safe for the bird immediately before hatching inside the eggshell.

The chemical solution is preferably injected into the inside of the eggshell by an automatic instrument. The automatic instrument holds the liquid injection needle element. On the other hand, a movable holding table is moved with a plurality of eggs held in a plurality of egg receiving recesses formed thereon. When each egg reaches a position below the forward end portion of the cannula 21, the egg receiving recess is moved upward by a predetermined distance. Then, the forward end portion of the cannula 21 is plunged into the eggshell. In this state, a predetermined amount of chemical solution is injected into the eggshell. After injection of the chemical solution, the egg receiving recess is moved downward. Next, another egg receiving recess is moved to the position below the cannula 21 and moved upward. Then, the chemical solution is injected into another egg in the similar manner.

Further, the liquid injection needle element according to this invention may be used to clean an organ by a cleaning liquid during surgical operation after the organ is incised and before the organ is sutured.

Further, the liquid injection needle element according to this invention may be used to clean a cavity during surgical operation after removing a lesion.

Although this invention has been described in conjunction with a few preferred embodiments thereof, this invention may be modified in various other manners within the scope of the appended claims.

## Claims

1. A liquid injection needle element comprising a cannula having a front part in a direction of a center axis with its forward end portion closed, the cannula having a plurality of nozzle orifices formed on the front part and spaced from one another so as to allow a liquid in the cannula to be jetted out of the cannula when the liquid is applied with pressure, wherein:
each of the nozzle orifices has an orifice axis intersecting the center axis;
each of the nozzle orifices having a first diameter in a first direction and a second diameter in a second direction intersecting the first direction, the first diameter ranging between 0.1 mm and 0.5 mm, the second diameter ranging between 0.1 mm and 0.3 mm;
the liquid being applied with pressure ranging between 19.613 x 10³ Pa and 196.133 x 10³ Pa to jet the liquid from the nozzle orifices in an angle range of 45 degrees towards the forward end portion with respect to the orifice axis.

2. The liquid injection needle element according to claim 1, wherein the nozzle orifices are formed at positions shifted in angle from one another around the center axis.

3. The liquid injection needle element according to claim 1 or 2, wherein the cannula is provided with a plurality of index marks formed on its outer surface at predetermined positions from the forward end portion in the direction of the center axis.

4. The liquid injection needle element according to any of claims 1 to 3, wherein each of the nozzle orifices is surrounded by a marginal wall surface coated with a resin layer.

5. A liquid injection needle element comprising a cannula having a front part in a direction of a center axis with its forward end portion closed, the cannula having a plurality of nozzle orifices formed on the front part and spaced from one another so as to allow a liquid in the cannula to be jetted out of the cannula when the liquid is applied with pressure, wherein:
the nozzle orifices are spaced from one another in the direction of the center axis, the cannula having a base extending along the center axis and a curved portion extending from one end of the base towards the front part of the cannula and bent around the center axis.

6. The liquid injection needle element according to claim 5, wherein each of the nozzle orifices has an orifice axis intersecting the center axis, each of the nozzle orifices having a first diameter in a first direction and a second diameter in a second direction intersecting the first direction, the first diameter ranging between 0.1 mm and 0.5 mm, the second diameter ranging between 0.1 mm and 0.3 mm, the liquid being applied with pressure ranging between 19.613 x 10³ Pa and 196.133 x 10³ Pa to jet the liquid from the nozzle orifices in an angle range of 45 degrees towards the forward end portion with respect to the orifice axis.

7. The liquid injection needle element according to claim 5 or 6, wherein the nozzle orifices are formed at positions shifted in angle from one another around the center axis.

8. The liquid injection needle element according to any of claims 5 to 7, wherein the cannula is provided with a plurality of index marks formed on its outer surface at predetermined positions from the forward end portion in the direction of the center axis.

9. The liquid injection needle element according to any of claims 5 to 8, wherein each of the nozzle orifices is surrounded by a marginal wall surface coated with a resin layer.

10. A method of producing a liquid injection needle element comprising a cannula having a front part in a direction of a center axis with its forward end portion closed, the cannula having a plurality of nozzle orifices formed on the front part and spaced from one another so as to allow a liquid in the cannula to be jetted out of the cannula when the liquid is applied with pressure, the method comprising the steps of:
preparing a tubular workpiece, facing a discharge wire to an outer surface of the workpiece in a direction intersecting the center axis of the workpiece, and moving the discharge wire to form the nozzle orifice by wire electric discharge;
moving the discharge wire in the direction of the center axis to successively form the nozzle orifices at a front part of the workpiece with a space kept from one another in the direction of the center axis; and
sealing and closing a forward end portion of the workpiece in the direction of the center axis.

11. The method according to claim 10, further comprising the step of bending a portion of the workpiece between the front part and a base opposite to the front part around the center axis of the workpiece to form a curved portion.

12. The method according to claim 10 or 11, wherein each of the nozzle orifices has an orifice axis intersecting the center axis, each of the nozzle orifices having a first diameter in a first direction and a second diameter in a second direction intersecting the first direction, the first diameter ranging between 0.1 mm and 0.5 mm, the second diameter ranging between 0.1 mm and 0.3 mm, the liquid being applied with pressure ranging between 19.613 x 10³ Pa and 196.133 x 10³ Pa to jet the liquid from the nozzle orifices in an angle range of 45 degrees towards the forward end portion with respect to the orifice axis.

13. The method according to any of claims 10 to 12, wherein one of the discharge wire and the workpiece is moved in the direction of the center axis after each nozzle orifice is formed, the workpiece being rotated around the center axis by a predetermined angle so that the nozzle orifices are formed at positions angularly shifted from one another around the center axis.

14. The method according to any of claims 10 to 13, wherein the cannula is provided with a plurality of index marks formed on its outer surface at predetermined positions from the forward end portion in the direction of the center axis.

15. The method according to any of claims 10 to 14, wherein each of the nozzle orifices is surrounded by a marginal wall surface coated with a resin layer.

16. The method according to any of claims 10 to 15, wherein, in the sealing step, an outer surface of the forward end portion of the cannula is formed into a semispherical shape by laser machining to close the forward end portion.
